# EUROPÄISCHE PATENTSCHRIFT

(11) **EP 2 099 453 B1**
(45) Veröffentlichungstag und Bekanntmachung des Hinweises auf die Patenterteilung: **06.09.2017**
(21) Anmeldenummer: 07819133.5
(22) Anmeldetag: 19.10.2007
(51) Int. Cl.: A61K 31/4365, A61K 31/52, A61K 31/5377, A61K 31/60, A61P 7/02, A61P 9/04, A61P 9/10, A61P 43/00

(54) **Kombinationstherapie substituierter Oxazolidinone**
Combination therapy of substituted oxazolidinones
Thérapie combinatoire à base d'oxazolidinone substituée

(30) Priorität: 02.11.2006 DE 102006051625
(43) Veröffentlichungstag der Anmeldung: 16.09.2009
(73) Patentinhaber: Bayer Intellectual Property GmbH, 40789 Monheim (DE)
(72) Erfinder: PERZBORN, Elisabeth, 42327 Wuppertal (DE)
(74) Vertreter: BIP Patents
(86) Internationale Anmeldenummer: PCT/EP2007/009068
(87) Internationale Veröffentlichungsnummer: WO 2008/052671

(56) Entgegenhaltungen:
- WO-A-2006/045756
- DE-A1- 10 129 725
- KUBITZA DAGMAR; BECKA MICHAEL; MUECK WOLFGANG; ZUEHLSDORF MICHAEL: "Safety, tolerability, pharmacodynamics, and pharmacokinetics of rivaroxaban - an oral, direct Factor Xa inhibitor - Are not affected by aspirin" JOURNAL OF CLINICAL PHARMACOLOGY, Bd. 46, Nr. 9, September 2006 (2006-09), Seiten 981-990, XP009098173
- COMMIT (CLOPIDOGREL AND METOPROLOL IN MYOCARDIAL INFARCTION TRIAL) COLLABORATIVE GROUP: "Addition of clopidogrel to aspirin in 45852 patients with acute myocardial infarction: randomised placebo-controlled trial" LANCET THE, LANCET LIMITED. LONDON, GB, Bd. 366, Nr. 9497, 5. November 2005 (2005-11-05), Seiten 1607-1621, XP005149889 ISSN: 0140-6736

## Beschreibung

Die vorliegende Erfindung betrifft eine Kombination von A) 5-Chloro-*N*-({(5*S*)-2-oxo-3-[4-(3-oxo-4-morpholinyl)phenyl]-1,3-oxazolidin-5-yl}methyl)-2-thiophencarboxamid mit B) Acetylsalizylsäure (Aspirin) und C) Clopidogrel, ein Verfahren zur Herstellung dieser Kombination und ihre Verwendung als Arzneimittel, insbesondere zur Prophylaxe und/oder Behandlung von thromboembolischen Erkrankungen.

Oxazolidinone der Formel (I) wirken insbesondere als selektive Inhibitoren des Blutgerinnungsfaktors Xa (FXa) und als Antikoagulantien (vergl. WO 01/47919). Kombinationen von FXa-Inhibitoren mit Plättchenaggregationshemmern, Antikoagulantien, Fibrinolytika, Lipidsenkern, Koronartherapeutika und/oder Vasodilatatoren sind beschrieben in WO 03/000256 und DE 10129725.

Eine antithrombotische Wirkung von Faktor Xa-Inhibitoren konnte in zahlreichen Tiermodellen (vgl. U. Sinha, P. Ku, J. Malinowski, B. Yan Zhu, RM. Scarborough, C K. Marlowe, PW. Wong, P. Hua Lin, SJ. Hollenbach, Antithrombotic and hemostatic capacity of factor Xa versus thrombin inhibitors in models of venous and arteriovenous thrombosis, European Journal of Pharmacology 2000, 395, 51-59; A. Betz, Recent advances in Factor Xa inhibitors, Expert Opin. Ther. Patents 2001, 11, 1007; K. Tsong Tan, A. Makin, G. YH Lip, Factor X inhibitors, Exp. Opin. Investig. Drugs 2003, 12, 799; J. Ruef, HA. Katus, New antithrombotic drugs on the horizon, Expert Opin. Investig. Drugs 2003, 12, 781; MM. Samama, Synthetic direct and indirect factor Xa inhibitors, Thrombosis Research 2002, 106, V267; ML. Quan, JM. Smallheer, The race to an orally active Factor Xa inhibitor, Recent advances, J. Current Opinion in Drug Discovery& Development 2004, 7, 460-469) sowie in klinischen Studien an Patienten (The Ephesus Study, blood 2000, Vol 96, 490a; The Penthifra Study, blood 2000, Vol 96, 490a; The Pentamaks Study, blood 2000, Vol 96, 490a-491a; The Pentathlon 2000 Study, blood 2000, Vol 96, 491a) nachgewiesen werden. Faktor Xa-Inhibitoren können deshalb bevorzugt eingesetzt werden in Arzneimitteln zur Prophylaxe und/oder Behandlung von thromboembolischen Erkrankungen.

Selektive FXa-Inhibitoren zeigen ein breites therapeutisches Fenster. In zahlreichen tierexperimentellen Untersuchungen konnte gezeigt werden, dass FXa-Inhibitoren in Thrombosemodellen eine antithrombotische Wirkung zeigen ohne, oder nur geringfügig, verlängernd auf Blutungszeiten zu wirken (vergl. R. J. Leadly, Coagulationfactor Xa inhibition: biological background and rationale, Curr Top Med Chem 2001; 1, 151-159).

Rivaroxaban (BAY 59-7939) ist ein neuartiger, in der klinischen Entwicklung befindlicher direkter Faktor Xa (FXa) Inhibitor, der zur Behandlung und Prävention von thromboembolischen Erkrankungen eingesetzt werden soll. In tierexperimentellen Untersuchungen (Perzborn, E, Srassburger J, Wilmen A, Pohlmann J, Roehrig S, Schlemmer KH, Straub A, In vitro and in vivo studies of the novel antithrombotic agent BAY 59-7939- an oral, direct Factor Xa inhibitor. JTH 2005; 3: 514-521) sowie in klinischen Studien (Kubitza D, Haas S, Novel factor Xa inhibitors for prevention and treatment of thromboembolic diseases. Expert Opin Investig Drugs 2006; 15: 843-855) wurde die antithrombotische Wirkung von Rivaroxaban gezeigt.

In klinischen Studien konnte belegt werden, dass die Therapie mit Hemmern der Plättchenaggregation wie Aspirin (Acetylsalizylsäure) (Antithrombotic Trialists Collaboration; Collaborative meta-analysis of randomized trials of antiplatelet therapy for prevention of death, myocardial infarction, and stroke in high risk patients. BMJ 2002; 324: 71-86), Clopidogrel (CAPRIE Steering Committee; A randomised, blinded, trial of clopidogrel versus aspirin in patients at risk of ischaemic events (CAPRIE). Lancet 1996; 348: 1329-39) und besonders deren Kombination (Peters RJ, Mehta SR, Fox KA et al. Effects of aspirin dose when used alone or in combination with clopidogrel in patients with acute coronary syndromes: observations from the Clopidogrel in Unstable angina to prevent Recurrent Events (Cure) study. Circulation 2003; 108: 1682-7; Chen ZM, Jiang LX, Chen YP et al. Addition of clopidogrel to aspirin in 45,852 patients with acute myocardial infarction: randomised placebo-controlled trial. Lancet 2005; 366: 1607-21) zu einer Verminderung von ischämischen Ereignissen (wie Myokardinfarkt, Hirnschlag) führt. Jedoch, trotz der offensichtlichen Verbesserung ist die Therapie mit Plättchenaggregationshemmen in ihrer Wirkung limitiert.

Es wurde nun überraschenderweise gefunden, dass die Kombination von 5-Chloro-*N*-({(5*S*)-2-oxo-3-[4-(3-oxo-4-morpholinyl)phenyl]-1,3-oxazolidin-5-yl}methyl)-2-thiophencarboxamid mit Acetylsalizylsäure und Clopidogrel, interessante Eigenschaften besitzt und für die Prophylaxe und/oder Behandlung von thromboembolischen Erkrankungen besser geeignet ist als die Einzelwirkstoffe alleine, die Kombination von 5-Chloro-*N*-({(5*S*)-2-oxo-3-[4-(3-oxo-4-morpholinyl)phenyl]-1,3-oxazolidin-5-yl}methyl)-2-thiophencarboxamid mit Acetylsalizylsäure, die Kombination von 5-Chloro-*N*-({(5*S*)-2-oxo-3-[4-(3-oxo-4-morpholinyl)phenyl]-1,3-oxazolidin-5-yl}methyl)-2-thiophencarboxamid mit Clopidogrel oder die Kombination von Acetylsalizylsäure und Clopidogrel.

Gegenstand der Erfindung ist daher die Kombination von
A) 5-Chloro-*N*-({(5*S*)-2-oxo-3-[4-(3-oxo-4-morpholinyl)phenyl]-1,3-oxazolidin-5-yl}methyl)-2-thiophencarboxamid (Rivaroxaban) der Formel 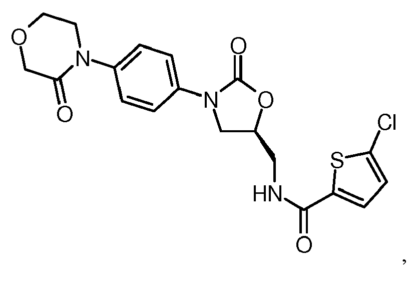 mit
B) Acetylsalizylsäure und
C) Clopidogrel.

Unter "Kombinationen" im Sinne der Erfindung werden nicht nur Darreichungsformen, die alle Komponenten enthalten (sog. Fixkombinationen), und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden.

Oxazolidinone sind beispielsweise Verbindungen der Formel (I) 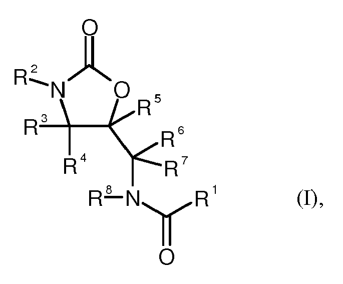 in welcher:
- R¹: für gegebenenfalls benzokondensiertes Thiophen (Thienyl) steht, das gegebenenfalls ein-oder mehrfach substituiert sein kann;
- R²: für einen beliebigen organischen Rest steht;
- R³, R⁴, R⁵, R⁶, R⁷ und R⁸: gleich oder verschieden sind und für Wasserstoff oder für (C₁-C₆)-Alkyl stehen
sowie deren pharmazeutisch verträglichen Salze, Hydrate und Prodrugs.

Oxazolidinone sind beispielsweise Verbindungen der Formel (I),
worin
- R¹: für gegebenenfalls benzokondensiertes Thiophen (Thienyl) steht, das gegebenenfalls ein- oder mehrfach substituiert sein kann durch einen Rest aus der Gruppe von Halogen; Cyano; Nitro; Amino; Aminomethyl; (C₁-C₈)-Alkyl, das gegebenenfalls seinerseits ein- oder mehrfach durch Halogen substituiert sein kann; (C₃-C₇)-Cycloalkyl; (C₁-C₈)-Alkoxy; Imidazolinyl; -C(=NH)NH₂; Carbamoyl; und Mono- und Di-(C₁-C₄)-alkyl-aminocarbonyl,
- R²: für eine der folgenden Gruppen steht:

A-,
A-M-,
D-M-A-,
B-M-A-,
B-,
B-M-,
B-M-B-,
D-M-B-,
wobei:
der Rest "A" für (C₆-C₁₄)-Aryl, vorzugsweise für (C₆-C₁₀)-Aryl, insbesondere für Phenyl oder Naphthyl, ganz besonders bevorzugt für Phenyl, steht;
der Rest "B" für einen 5- oder 6-gliedrigen aromatischen Heterocyclus steht, der bis zu 3 Heteroatome und/oder Hetero-Kettenglieder, insbesondere bis zu 2 Heteroatome und/oder Hetero-Kettenglieder, aus der Reihe S, N, NO (N-Oxid) und O enthält;
der Rest "D" für einen gesättigten oder teilweise ungesättigten, mono- oder bicyclischen, gegebenenfalls benzokondensierten 4- bis 9-gliedrigen Heterocyclus steht, der bis zu drei Heteroatome und/oder Hetero-Kettenglieder aus der Reihe S, SO, SO₂, N, NO (N-Oxid) und O enthält;
der Rest "M" für -NH-, -CH₂-, -CH₂CH₂-, -O-, -NH-CH₂-, -CH₂-NH-, -OCH₂-, -CH₂O-, -CONH-, -NHCO-, -COO-, -OOC-, -S-, -SO₂- oder für eine kovalente Bindung steht;
wobei
die zuvor definierten Gruppen "A", "B" und "D" jeweils gegebenenfalls ein- oder mehrfach substituiert sein können mit einem Rest aus der Gruppe von Halogen; Trifluormethyl; Oxo; Cyano; Nitro; Carbamoyl; Pyridyl; (C₁-C₆)-Alkanoyl; (C₃-C₇)-Cycloalkanoyl; (C₆-C₁₄)-Arylcarbonyl; (C₅-C₁₀)-Heteroarylcarbonyl; (C₁-C₆)-Alkanoyloxymethyloxy; (C₁-C₄)-Hydroxyalkylcarbonyl; -COOR²⁷; -SO₂R²⁷; -C(NR²⁷R²⁸)=NR²⁹; -CONR²⁸R²⁹; -SO₂NR²⁸R²⁹; -OR³⁰; -NR³⁰R³¹, (C₁-C₆)-Alkyl und (C₃-C₇)-Cycloalkyl,
wobei (C₁-C₆)-Alkyl und (C₃-C₇)-Cycloalkyl ihrerseits gegebenenfalls substituiert sein können durch einen Rest aus der Gruppe von Cyano; -OR²⁷; -NR²⁸R²⁹; -CO(NH)ᵥ(NR²⁷R²⁸) und -C(NR²⁷R²⁸)=NR²⁹,
wobei:

v entweder 0 oder 1 bedeutet und
R²⁷, R²⁸ und R²⁹ gleich oder verschieden sind und unabhängig voneinander Wasserstoff, (C₁-C₄)-Alkyl, (C₃-C₇)-Cycloalkyl, (C₁-C₄)-Alkanoyl, Carbamoyl, Trifluormethyl, Phenyl oder Pyridyl bedeuten,
und/oder
R²⁷ und R²⁸ bzw. R²⁷ und R²⁹ zusammen mit dem Stickstoffatom, an das sie gebunden sind, einen gesättigten oder teilweise ungesättigten 5- bis 7-gliedrigen Heterocyclus mit bis zu drei, vorzugsweise bis zu zwei gleichen oder unterschiedlichen Hetero-atomen aus der Gruppe von N, O und S bilden, und
R³⁰ und R³¹ gleich oder verschieden sind und unabhängig voneinander Wasserstoff, (C₁-C₄)-Alkyl, (C₃-C₇)-Cycloalkyl, (C₁-C₄)-Alkylsulfonyl, (C₁-C₄)-Hydroxyalkyl, (C₁-C₄)-Aminoalkyl, Di-(C₁-C₄)-alkylamino-(C₁-C₄)-alkyl, -CH₂C(NR²⁷R²⁸)=NR²⁹ oder -COR³³ bedeuten,
wobei
R³³ (C₁-C₆)-Alkoxy, (C₁-C₄)-Alkoxy-(C₁-C₄)-alkyl, (C₁-C₄)-Alkoxycarbonyl-(C₁-C₄)-alkyl, (C₁-C₄)-Aminoalkyl, (C₁-C₄)-Alkoxycarbonyl, (C₁-C₄)-Alkanoyl-(C₁-C₄)-alkyl, (C₃-C₇)-Cycloalkyl, (C₂-C₆)-Alkenyl, (C₁-C₈)-Alkyl, das gegebenenfalls durch Phenyl oder Acetyl substituiert sein kann, (C₆-C₁₄)-Aryl, (C₅-C₁₀)-Heteroaryl, Trifluormethyl, Tetrahydrofuranyl oder Butyrolacton bedeutet,
- R³, R⁴, R⁵, R⁶, R⁷ und R⁸: gleich oder verschieden sind und für Wasserstoff oder für (C₁-C₆)-Alkyl stehen
und deren pharmazeutisch verträglichen Salze, Hydrate und Prodrugs.

Ebenfalls ganz besonders bevorzugt ist hierbei die Verbindung mit der folgenden Formel 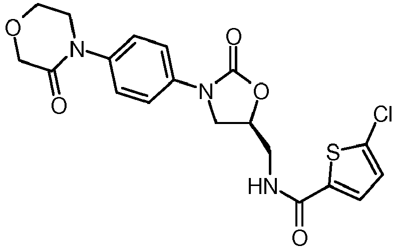 und ihre pharmazeutisch verträglichen Salze, Hydrate und Prodrugs.

Bislang sind Oxazolidinone im wesentlichen nur als Antibiotika, vereinzelt auch als MAO-Hemmer und Fibrinogen-Antagonisten beschrieben (Übersicht: Riedl, B., Endermann, R., Exp. Opin. Ther. Patents 1999, 9 (5), 625), wobei für die antibakterielle Wirkung eine kleine 5-[Acyl-aminomethyl]-gruppe (bevorzugt 5-[Acetyl-aminomethyl]) essentiell zu sein scheint.

Substituierte Aryl- und Heteroarylphenyloxazolidinone, bei denen an das N-Atom des Oxazolidinonrings ein ein- oder mehrfach substituierte Phenylrest gebunden sein kann und die in der 5-Position des Oxazolidinonrings einen unsubstituierten N-Methyl-2-thiophencarboxamid-Rest aufweisen können, sowie ihre Verwendung als antibakteriell wirkende Substanzen sind bekannt aus den U.S.-Patentschriften US-A-5 929 248, US-A-5 801 246, US-A-5 756 732, US-A-5 654 435, US-A-5 654 428 und US-A-5 565 571.

Darüber hinaus sind benzamidinhaltige Oxazolidinone als synthetische Zwischenstufen bei der Synthese von Faktor Xa-Inhibitoren bzw. Fibrinogenantagonisten bekannt (WO-A-99/31092, EP-A-623615).

Die Verbindungen der Formel (I) können in Abhängigkeit von dem Substitutionsmuster in stereoisomeren Formen, die sich entweder wie Bild und Spiegelbild (Enantiomere) oder die sich nicht wie Bild und Spiegelbild (Diastereomere) verhalten, existieren. Umfasst sind sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen der Formel (I) in tautomeren Formen vorliegen. Dies ist dem Fachmann bekannt, und derartige Verbindungen sind ebenfalls umfasst.

Physiologisch unbedenkliche, d.h. pharmazeutisch verträgliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Trifluoressigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Als pharmazeutisch verträgliche Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin oder Methylpiperidin.

Als "Hydrate" werden solche Formen der Verbindungen der obigen Formel (I) bezeichnet, welche in festem oder flüssigem Zustand durch Hydratation mit Wasser eine Molekül-Verbindung (Solvat) bilden. In den Hydraten sind die Wassermoleküle nebenvalent durch zwischenmolekulare Kräfte, insbesondere Wasserstoff-Brückenbindungen angelagert. Feste Hydrate enthalten Wasser als sogenanntes Kristall-Wasser in stöchiometrischen Verhältnissen, wobei die Wassermoleküle hinsichtlich ihres Bindungszustands nicht gleichwertig sein müssen. Beispiele für Hydrate sind Sesquihydrate, Monohydrate, Dihydrate oder Trihydrate. Gleichermaßen kommen auch die Hydrate von Salzen der erfindungsgemäßen Verbindungen in Betracht.

Als "Prodrugs" werden solche Formen der Verbindungen der obigen Formel (I) bezeichnet, welche selbst biologisch aktiv oder inaktiv sein können, jedoch in die entsprechende biologisch aktive Form überführt werden können (beispielsweise metabolisch, solvolytisch oder auf andere Weise).

Halogen steht für Fluor, Chlor, Brom und Iod. Bevorzugt sind Chlor oder Fluor.

(C₁-C₈)-Alkyl steht für einen geradkettigen oder verzweigten Alkylrest mit 1 bis 8 Kohlenstoffatomen. Beispielsweise seien genannt: Methyl, Ethyl, n-Propyl, Isopropyl, n-Butyl, Isobutyl, tert.-Butyl, n-Pentyl und n-Hexyl. Aus dieser Definition leiten sich analog die entsprechenden Alkylgruppen mit weniger Kohlenstoffatomen wie z.B. (C₁-C₆)-Alkyl und (C₁-C₄)-Alkyl ab. Im allgemeinen gilt, dass (C₁-C₄)-Alkyl bevorzugt ist.

Aus dieser Definition leitet sich auch die Bedeutung des entsprechenden Bestandteils anderer komplexerer Substituenten ab wie z.B. bei Alkylsulfonyl, Hydroxyalkyl, Hydroxyalkylcarbonyl, Alkoxy-alkyl, Alkoxycarbonyl-alkyl, Alkanoylalkyl, Aminoalkyl oder Alkylaminoalkyl.

(C₃-C₇-Cycloalkyl steht für einen cyclischen Alkylrest mit 3 bis 7 Kohlenstoffatomen. Beispielsweise seien genannt: Cyclopropyl, Cyclobutyl, Cyclopentyl, Cyclohexyl oder Cycloheptyl. Aus dieser Definition leiten sich analog die entsprechenden Cycloalkylgruppen mit weniger Kohlenstoffatomen wie z.B. (C₃-C₅)-Cycloalkyl ab. Bevorzugt sind Cyclopropyl, Cyclopentyl und Cyclohexyl.

Aus dieser Definition leitet sich auch die Bedeutung des entsprechenden Bestandteils anderer komplexerer Substituenten ab wie z.B. Cycloalkanoyl.

(C₂-C₆)-Alkenyl steht für einen geradkettigen oder verzweigten Alkenylrest mit 2 bis 6 6 Kohlenstoffatomen. Bevorzugt ist ein geradkettiger oder verzweigter Alkenylrest mit 2 bis 4 Kohlenstoffatomen. Beispielsweise seien genannt: Vinyl, Allyl, Isopropenyl und n-But-2-en-1-yl.

(C₁-C₈)-Alkoxy steht für einen geradkettigen oder verzweigten Alkoxyrest mit 1 bis 8 Kohlenstoffatomen. Beispielsweise seien genannt: Methoxy, Ethoxy, n-Propoxy, Isopropoxy, n-Butoxy, Isobutoxy, tert.-Butoxy, n-Pentoxy, n-Hexoxy, n-Heptoxy und n-Oktoxy. Aus dieser Definition leiten sich analog die entsprechenden Alkoxygruppen mit weniger Kohlenstoffatomen wie z.B. (C₁-C₆)-Alkoxy und (C₁-C₄)-Alkoxy ab. Im allgemeinen gilt, dass (C₁-C₄)-Alkoxy bevorzugt ist.

Aus dieser Definition leitet sich auch die Bedeutung des entsprechenden Bestandteils anderer komplexerer Substituenten ab wie z.B. Alkoxy-alkyl, Alkoxycarbonyl-alkyl und Alkoxycarbonyl.

Mono- oder Di-(C₁-C₄)-Alkylaminocarbonyl steht für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 4 Kohlenstoffatomen aufweist. Beispielsweise seien genannt: Methylamino, Ethylamino, n-Propylamino, Isopropylamino, t-Butylamino, *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino und *N*-t-Butyl-*N*-methylamino.

(C₁-C₆)-Alkanoyl steht für einen geradkettigen oder verzweigten Alkylrest mit 1 bis 6 Kohlenstoffatomen, der in der 1-Position ein doppelt gebundenes Sauerstoffatom trägt und über die 1-Position verknüpft ist. Beispielsweise seien genannt: Formyl, Acetyl, Propionyl, n-Butyryl, i-Butyryl, Pivaloyl, n-Hexanoyl. Aus dieser Definition leiten sich analog die entsprechenden Alkanoylgruppen mit weniger Kohlenstoffatomen wie z.B. (C₁-C₅)-Alkanoyl, (C₁-C₄)-Alkanoyl und (C₁-C₃)-Alkanoyl ab. Im allgemeinen gilt, dass (C₁-C₃)-Alkanoyl bevorzugt ist.

Aus dieser Definition leitet sich auch die Bedeutung des entsprechenden Bestandteils anderer komplexerer Substituenten ab wie z.B. Cycloalkanoyl und Alkanoylalkyl.

(C₃-C₇)-Cycloalkanoyl steht für einen wie zuvor definierten Cycloalkylrest mit 3 bis 7 Kohlenstoffatomen, der über eine Carbonylgruppe verknüpft ist.

(C₁-C₆)-Alkanoyloxymethyloxy steht für einen geradkettigen oder verzweigten Alkanoyloxymethyloxy-Rest mit 1 bis 6 Kohlenstoffatomen. Beispielsweise seien genannt: Acetoxymethyloxy, Propionoxymethyloxy, n-Butyroxymethyloxy, i-Butyroxymethyloxy, Pivaloyloxymethyloxy, n-Hexanoyloxymethyloxy. Aus dieser Definition leiten sich analog die entsprechenden Alkanoyloxymethyloxy-Gruppen mit weniger Kohlenstoffatomen wie z.B. (C₁-C₃)-Alkanoyloxymethyloxy ab. Im allgemeinen gilt, dass (C₁-C₃)-Alkanoyloxymethyloxy bevorzugt ist.

(C₆-C₁₄)-Aryl steht für einen aromatischen Rest mit 6 bis 14 Kohlenstoffatomen. Beispielsweise seien genannt: Phenyl, Naphthyl, Phenanthrenyl und Anthracenyl. Aus dieser Definition leiten sich analog die entsprechenden Arylgruppen mit weniger Kohlenstoffatomen wie z.B. (C₆-C₁₀)-Aryl ab. Im allgemeinen gilt, dass (C₆-C₁₀)-Aryl bevorzugt ist.

Aus dieser Definition leitet sich auch die Bedeutung des entsprechenden Bestandteils anderer komplexerer Substituenten ab wie z.B. Arylcarbonyl.

(C₅-C₁₀)-Heteroaryl oder ein 5- bis 10-gliedriger aromatischer Heterocyclus mit bis zu 3 Heteroatomen und/oder Heterokettengliedern aus der Reihe S, O, N und/oder NO (N-Oxid) steht für einen mono- oder bicyclischen Heteroaromaten, der über ein Ringkohlenstoffatom des Heteroaromaten, gegebenenfalls auch über ein Ringstickstoffatom des Heteroaromaten, verknüpft ist. Beispielsweise seien genannt: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. Aus dieser Definition leiten sich analog die entsprechenden Heterocyclen mit geringerer Ringgröße wie z.B. 5- oder 6-gliedrige aromatische Heterocyclen ab. Im allgemeinen gilt, dass 5- oder 6-gliedrige aromatische Heterocyclen wie z.B. Pyridyl, Pyridyl-N-oxid, Pyrimidyl, Pyridazinyl, Furyl und Thienyl bevorzugt sind.

Aus dieser Definition leitet sich auch die Bedeutung des entsprechenden Bestandteils anderer komplexerer Substituenten ab wie z.B. (C₅-C₁₀)-Heteroarylcarbonyl.

Ein 3- bis 9-gliedriger gesättigter oder teilweise ungesättigter, mono- oder bicyclischer, gegebenenfalls benzokondensierter Heterocyclus mit bis zu 3 Heteroatomen und/oder Heterokettengliedern aus der Reihe S, SO, SO₂, N, NO (N-Oxid) und/oder O steht für einen Heterocyclus, der eine oder mehrere Doppelbindungen enthalten kann, der mono- oder bicyclisch sein kann, bei dem an zwei benachbarte Ringkohlenstoffatomen ein Benzolring ankondensiert sein kann und der über ein Ringkohlenstoffatom oder ein Ringstickstoffatom verknüpft ist. Beispielsweise seien genannt: Tetrahydrofuryl, Pyrrolidinyl, Pyrrolinyl, Piperidinyl, 1,2-Dihydropyridinyl, 1,4-Dihydropyridinyl, Piperazinyl, Morpholinyl, Morpholinyl-N-oxid, Thiomorpholinyl, Azepinyl, 1,4-Diazepinyl und Cyclohexyl. Bevorzugt sind Piperidinyl, Morpholinyl und Pyrrolidinyl.

Aus dieser Definition leiten sich analog die entsprechenden Cyclen mit geringerer Ringgröße wie z.B. 5- bis 7-gliedrige Cyclen ab.

Die Verbindungen der Formel (I) können hergestellt werden wie in WO 01/47919 beschrieben.

Eine bevorzugte Verbindung A) der Formel (I) für den Einsatz in Kombinationen ist 5-Chloro-*N-*({(5*S*)-2-oxo-3-[4-(3-oxo-4-morpholinyl)phenyl]-1,3-oxazolidin-5-yl}methyl)-2-thiophencarbox-amid (Rivaroxaban), die Verbindung aus Beispiel 44.

Eine besonders bevorzugte Verbindung C) eines ADP Rezeptor Antagonisten, insbesondere eines P₂Y₁₂ Rezeptor Blockers, ist Clopidogrel (Plavix).

Ein niedrig dosierter FXa Inhibitor, wie Rivaroxaban (Beispiel 44), in der Kombination mit Acetylsalizylsäure und einem ADP Rezeptor Antagonisten, wie Clopidogrel, führt zu einer potenten synergistischen antithrombotischen Wirkung und ist der Wirkung der Kombination von Oxazolidinonen der Formel (I) mit Acetylsalizylsäure oder der Kombination von Oxazolidinonen der Formel (I) mit einem ADP Rezeptor Antagonisten sowie der Kombination von Acetylsalizylsäure und einem ADP Rezeptor Antagonisten alleine überlegen. Rivaroxaban, gegeben in Dosierungen, die in alleiniger Anwendung keine antithrombotische Wirkung zeigen, führt in Kombination mit Acetylsalizylsäure und Clopidogrel, einem P₂Y₁₂ Rezeptor Blocker (ADP Rezeptor Antagonist), zu einer erheblichen Wirksteigerung der antithrombotischen Wirkung der Plättchenaggregationshemmer in einem Thrombosemodel.

Die erfindungsgemäßen Kombinationen sind insbesondere zur Behandlung und/oder Prophylaxe von thromboembolischen Erkrankungen geeignet.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken, thrombotischer und thromboembolischer Hirnschlag.

Die erfindungsgemäßen Kombinationen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, sowie zur Prävention und Behandlung von kardiogenen Thromboembolien, wie beispielsweise Hirn-Ischämien, Schlaganfall und systemischen Thromboembolien und Ischämien, bei Patienten mit Herzklappen-Erkrankungen oder mit künstlichen Herzklappen.

Darüber hinaus sind die erfindungsgemäßen Kombinationen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Kombinationen auch für die Prophylaxe und/oder Behandlung von atherosklerotischen Gefäßerkrankungen und entzündlichen Erkrankungen wie Rheumatische Erkrankungen des Bewegungsapparats in Betracht, darüber hinaus ebenso für die Prophylaxe und/oder Behandlung der Alzheimer'schen Erkrankung.

Außerdem können die erfindungsgemäßen Kombinationen zur Inhibition des Tumorwachstums und Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die einzelnen Kombinationswirkstoffe sind literaturbekannt und größtenteils kommerziell erhältlich. Sie können gegebenenfalls, ebenso wie Oxazolidinone der Formel (I), in subtherapeutisch wirksamen Dosen eingesetzt werden.

Für die Applikation der erfindungsgemäßen Kombinationen kommen alle üblichen Applikationsformen in Betracht. Vorzugsweise erfolgt die Applikation oral, lingual, sublingual, bukkal, rektal, topical oder parenteral (d.h. unter Umgehung des Intestinaltraktes, also intravenös, intraarteriell, intrakardial, intrakutan, subkutan, transdermal, intraperitoneal oder intramuskulär).

Zur vorliegenden Erfindung gehören pharmazeutische Zubereitungen, die neben nicht-toxischen, inerten pharmazeutisch geeigneten Hilfs- und/oder Trägerstoffen eine oder mehrere erfindungsgemäße Kombinationen enthalten oder die aus einer erfindungsgemäßen Kombination bestehen, sowie Verfahren zur Herstellung dieser Zubereitungen.

Die erfindungsgemäßen Kombinationen sollen in den oben aufgeführten pharmazeutischen Zubereitungen in einer Konzentration von etwa 0,1 bis 99,5, vorzugsweise etwa 0,5 bis 95 Gew.-% der Gesamtmischung vorhanden sein.

Die oben aufgeführten pharmazeutischen Zubereitungen können außer den erfindungsgemäßen Kombinationen auch weitere pharmazeutische Wirkstoffe enthalten.

Die Herstellung der oben aufgeführten pharmazeutischen Zubereitungen kann in üblicher Weise nach bekannten Methoden erfolgen, z.B. durch Mischen des Wirkstoffes oder der Wirkstoffe mit dem oder den Trägerstoffen.

Im allgemeinen hat es sich als vorteilhaft erwiesen, die erfindungsgemäßen Kombinationen in Gesamtmengen von etwa 0,001 bis 100 mg/kg, vorzugsweise etwa 0,01 bis 100 mg/kg, insbesondere etwa 0,1 bis 10 mg/kg Körpergewicht je 24 Stunden, gegebenenfalls in Form mehrerer Einzelgaben, zur Erzielung der gewünschten Ergebnisse zu verabreichen.

Trotzdem kann es gegebenenfalls erforderlich sein, von den zuvor genannten Mengen abzuweichen, und zwar in Abhängigkeit vom Körpergewicht, von der Art des Applikationsweges, der Art und Schwere der Erkrankung, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Es kann beispielsweise im Falle der Applikation größerer Mengen empfehlenswert sein, diese über den Tag zu verteilen, und zwar entweder in mehreren Einzelgaben oder als Dauerinfusion.

Weiterer Gegenstand der Erfindung sind daher die oben definierten Kombinationen zur Prophylaxe und/oder Behandlung von Erkrankungen.

Weiterer Gegenstand der Erfindung sind Arzneimittel, enthaltend mindestens eine der oben definierten Kombinationen und gegebenenfalls weitere pharmazeutische Wirkstoffe.

Weiterer Gegenstand der Erfindung ist die Verwendung der oben definierten Kombinationen zur Herstellung von Arzneimitteln zur Prophylaxe und/oder Behandlung der oben beschriebenen Erkrankungen, vorzugsweise von thromboembolischen Erkrankungen, insbesondere von Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken, thrombotischer und thromboembolischer Hirnschlag

Die Prozentangaben der nachfolgenden Beispiele beziehen sich jeweils auf das Gewicht; Teile sind Gewichtsteile.

### Beispiele

### A Bewertung der physiologischen Wirksamkeit

### 1. Physiologische Wirksamkeit der Verbindungen der Formel (I)

Die Verbindungen der Formel (I) wirken insbesondere als selektive Inhibitoren des Blutgerinnungsfaktors Xa und hemmen nicht oder erst bei deutlich höheren Konzentrationen auch andere Serinproteasen wie Thrombin, Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Thrombin, Plasmin und Trypsin, um das 100-fache, vorzugsweise um das 500-fache, insbesondere um das 1.000-fache, kleiner sind, wobei bezüglich der Testmethoden für die Selektivität Bezug genommen wird auf die im folgenden beschriebenen Testmethoden der Beispiele A-1) a.1) und a.2).

Die besonders vorteilhaften biologischen Eigenschaften der Verbindungen der Formel (I) können durch folgende Methoden festgestellt werden.

### a) Testbeschreibung (in vitro)

### a.1) Messung der Faktor Xa-Hemmung

Die enzymatische Aktivität von humanem Faktor Xa (FXa) wurde über die Umsetzung eines für den FXa-spezifischen chromogenen Substrats gemessen. Dabei spaltet der Faktor Xa aus dem chromogenen Substrat p-Nitroanilin ab. Die Bestimmungen wurden wie folgt in Mikrotiterplatten durchgeführt.

Die Prüfsubstanzen wurden in unterschiedlichen Konzentrationen in DMSO gelöst und für 10 Minuten mit humanem FXa (0,5 mmol/l gelöst in 50 mmol/l Tris-Puffer [C,C,C-Tris(hydroxymethyl)-aminomethan], 150 mmol/l NaCl, 0,1 % BSA (bovine serum albumine), pH = 8,3) bei 25°C inkubiert. Als Kontrolle dient reines DMSO. Anschließend wurde das chromogene Substrat (150 µmol/l Pefachrome^{®} FXa von der Firma Pentapharm) hinzugefügt. Nach 20 Minuten Inkubationsdauer bei 25°C wurde die Extinktion bei 405 nm bestimmt. Die Extinktionen der Testansätze mit Prüfsubstanz wurden mit den Kontrollansätzen ohne Prüfsubstanz verglichen und daraus die IC₅₀-Werte berechnet.

### a.2) Bestimmung der Selektivität

Zum Nachweis der selektiven FXa-Inhibition wurden die Prüfsubstanzen auf ihre Hemmung anderer humaner Serinproteasen wie Thrombin, Trypsin, Plasmin hin untersucht. Zur Bestimmung der enzymatischen Aktivität von Thrombin (75 mU/ml), Trypsin (500 mU/ml) und Plasmin (3,2 nmol/l) wurden diese Enzyme in Tris-Puffer (100 mmol/l, 20 mmol/l CaCl₂, pH = 8,0) gelöst und für 10 Minuten mit Prüfsubstanz oder Lösungsmittel inkubiert. Anschließend wurde durch Zugabe der entsprechenden spezifischen chromogenen Substrate (Chromozym Thrombin^{®} von der Firma Boehringer Mannheim, Chromozym Trypsin^{®} von der Firma Boehringer Mannheim, Chromozym Plasmin^{®} von der Firma Boehringer Mannheim) die enzymatische Reaktion gestartet und die Extinktion nach 20 Minuten bei 405 nm bestimmt. Alle Bestimmungen wurden bei 37°C durchgeführt. Die Extinktionen der Testansätze mit Prüfsubstanz wurden mit den Kontrollproben ohne Prüfsubstanz verglichen und daraus die IC₅₀-Werte berechnet.

### a.3) Bestimmung der antikoagulatorischen Wirkung

Die antikoagulatorische Wirkung der Prüfsubstanzen wurde in vitro in Humanplasma bestimmt. Dazu wurde Humanblut unter Verwendung einer 0,11 molaren Natriumcitrat-Lösung als Vorlage in einem Mischungsverhältnis Natriumcitrat/Blut 1/9 abgenommen. Das Blut wurde unmittelbar nach der Abnahme gut gemischt und 10 Minuten bei ca. 2000 g zentrifugiert. Der Überstand wurde abpipettiert. Die Prothrombinzeit (PT, Synonyme: Thromboplastinzeit, Quick-Test) wurde in Gegenwart variierender Konzentrationen an Prüfsubstanz oder dem entsprechenden Lösungsmittel mit einem handelsüblichen Testkit (Neoplastin^{®} von der Firma Boehringer Mannheim) bestimmt. Die Testverbindungen wurden 10 Minuten bei 37°C mit dem Plasma inkubiert. Anschließend wurde durch Zugabe von Thromboplastin die Gerinnung ausgelöst und der Zeitpunkt des Gerinnungseintritts bestimmt. Es wurde die Konzentration an Prüfsubstanz ermittelt, die eine Verdoppelung der Prothrombinzeit bewirkt.

### 2. Physiologische Wirksamkeit der Kombinationen von Verbindungen der Formel (I)

### a) Bestimmung der antithrombotischen Wirkung in einem arteriovenösen Shunt Model in Ratten

Nüchterne männliche Ratten (Stamm: HSD CPB:WU) wurden durch intraperitoneale Gabe einer Rompun/Ketavet Lösung narkotisiert (12mg/kg/50mg/kg). Thrombe wurden in einem arteriovenösen Shunt, in dem ein thrombogener Faden eingebunden war, in Anlehnung an die von PC Wong et al. (Wong PC, Crain EJ, Nguan O, Watson CA, Racanelli A. Antithrombotic actions of selective inhibitors of blood coagulation factor Xa in rat models of thrombosis Thrombosis Research 1996; 83: 117-126) beschriebene Methode erzeugt. Dazu wurden die linke Vena jugularis und die rechte Arteria carotis freipräpariert. In die Arterie wurde ein 8 cm langer Polyethylenkatheter (PE60, Becton-Dickinson), gefolgt von einem 6 cm langen Tygonschlauch (R-3606, ID 3.2mm, Kronlab), der einen aufgerauten, thrombogenen und zu einer Doppelschlinge gelegten Nylonfaden (60 x 0,26 mm, Berkley Trilene) enthielt, eingebunden. In die Vena jugularis wurde ein 2 cm langer Polyethylenkatheter (PE60, Becton-Dickinson) eingebunden und über einen 6 cm langen Polyethylenkatheter (PE160, Becton-Dickinson) mit dem Tygonschlauch verbunden. Die Schläuche wurden mit physiologischer Kochsalzlösung vor Öffnung des Shunts gefüllt. Der extrakorporale Kreislauf wurde 15 Minuten lang aufrechterhalten. Dann wurde der Shunt entfernt, und der Nylonfaden mit dem Thrombus sofort gewogen. Das Leergewicht des Nylonfadens war vor Versuchsbeginn ermittelt worden. Der FXa-Inhibitor (wie Rivaroxaban) wurde den Tieren intravenös und Acetylsalizylsäure (ASS) und / oder ADP-Rezeptorantagonist (wie Clopidogrel) oral mittels Schlundsonde vor Anlegung des extrakorporalen Kreislaufs verabreicht.

**Tabelle 1: Synergistische antithrombotische Wirkung der Kombination eines Oxazolidinons der Formel (I) mit Acetylsalizylsäure und einem ADP-Rezeptorantagonisten**

| **Substanz** | **Beispiel 44** | **Clopi + ASS** | **Beispiel 44 + Clopi + ASS** |
|---|---|---|---|
| [mg/kg] | 0.01 iv | 1 po + 3 po | 0.01 iv + 1 po + 3 po |
| Thrombus Reduktion [%] | 18 | 20 | 43 |
| | p> 0.05 | p> 0.05 | P< 0.001 |
| | kein Effekt | kein Effekt | Wirkung |
| | | | |

| **Substanz** | **Beispiel 44** | **Clopi + ASS** | **Beispiel 44 + Clopi + ASS** |
|---|---|---|---|
| [mg/kg] | 0.03 iv | 1 po + 3 po | 0.03 iv + 1 po + 3 po |
| Thrombusgewichtsreduction [%] | 20 | 26 | 43 |
| | p> 0.05 | p< 0.05 | P< 0.001 |
| | kein Effekt | schwache Wirkung | Wirkung |

| | | | |
|---|---|---|---|
| Clopi = Clopidogrel, ASS = Acetylsalizylsäure (Aspirin) | | | |

Wie in Tabelle 1 gezeigt, wird mit der Kombination von Rivaroxaban (Beispiel 44) mit Acetylsalizylsäure und einem ADP Rezeptor Blocker, wie Clopidogrel, eine synergistische Wirkung erzielt, d. h., die drei Komponenten verstärken sich gegenseitig in ihrer Wirkung. In der Einzeldosierung ist Rivaroxaban (Beispiel 44) unwirksam und die Kombination der beiden Aggregationshemmer ist auch unwirksam bzw. nur sehr schwach wirksam. Die Kombination der drei Verbindungen führt dagegen zu einer hoch signifikanten Verminderung des Thrombusgewichts. Durch die Kombination eines Oxazolidinons der Fornmal (I) mit Acetylsalizylsäure und einem ADP Rezeptor Blocker kann daher die antithrombotische Therapie erheblich verbessert werden.

### B Herstellungbeispiele

### Ausgangsverbindungen

Die Darstellung von 3-Morpholinon wird in US 5 349 045 beschrieben.

Die Darstellung von N-(2,3-Epoxypropyl)phthalimid wird in J.-W. Chern et al. Tetrahedron Lett. 1998,39,8483 beschrieben.

Die substituierten Aniline können erhalten werden, indem man z.B. 4-Fluornitrobenzol, 2,4-Difluornitrobenzol oder 4-Chlornitrobenzol mit den entsprechenden Aminen oder Amiden in Gegenwart einer Base umsetzt. Dies kann auch unter Verwendung von Pd-Katalysatoren wie Pd(OAc)₂/DPPF/NaOt-Bu (Tetrahedron Lett. 1999,40,2035) oder Kupfer (Renger, Synthesis 1985,856; Aebischer et al., Heterocycles 1998,48,2225) geschehen. Genauso können Halogenaromaten ohne Nitrogruppe zunächst in die entsprechenden Amide umgewandelt werden, um sie anschließend in 4-Stellung zu nitrieren (US3279880).

### I. 4-(4-Morpholin-3-onyl)nitrobenzol

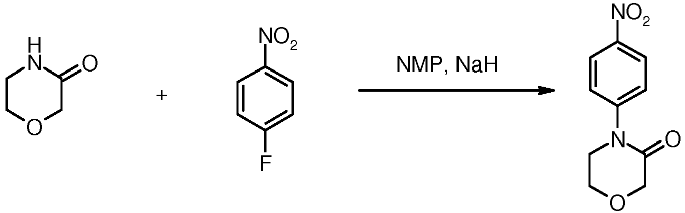

In 21 N-Methylpyrrolidon (NMP) werden 2 mol (202 g) Morpholin-3-on (E. Pfeil, U. Harder, Angew. Chem. 79, 1967, 188) gelöst. Über einen Zeitraum von 2 h erfolgt nun portionsweise die Zugabe von 88 g (2,2 mol) Natriumhydrid (60% in Paraffin). Nach Beendigung der Wasserstoffentwicklung werden unter Kühlung bei Raumtemperatur 282 g (2 mol) 4-Fluornitrobenzol innerhalb von 1 h zugetropft und das Reaktionsgemisch über Nacht nachgerührt. Im Anschluss werden bei 12 mbar und 76°C 1,7 l des Flüssigkeitsvolumens abdestilliert, der Rückstand auf 21 Wasser gegossen und dieses Gemisch zweimal mit je 11 Ethylacetat extrahiert. Nach dem Waschen der vereinigten organischen Phasen mit Wasser wird über Natriumsulfat getrocknet und das Lösemittel im Vakuum abdestilliert. Die Reinigung erfolgt durch Chromatographie an Kieselgel mit Hexan/Ethylacetat (1:1) und nachfolgende Kristallisation aus Ethylacetat. Das Produkt fällt mit 78 g als farbloser bis bräunlicher Feststoff in 17,6 % d. Th. an.
¹H-NMR (300 MHz, CDCl₃): 3,86 (m, 2 H, C*H*₂CH₂), 4,08 (m, 2 H, CH₂C*H*₂), 4,49 (s, 2 H, C*H*₂CO), 7,61 (d, 2 H, ³*J*=8,95 Hz, C*H*CH), 8,28 (d, 2 H, ³*J*=8,95 Hz, CHC*H*)
MS (r.I.%) = 222 (74, M⁺˙), 193 (100), 164 (28), 150 (21), 136 (61), 117 (22), 106 (24), 90 (37), 76 (38), 63 (32), 50 (25)

### II. 4-(4-Morpholin-3-onyl)anilin

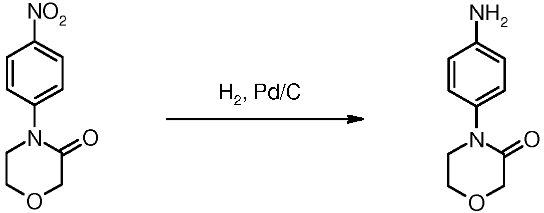

In einem Autoklaven werden 63 g (0,275 mol) 4-(4-Morpholin-3-onyl)nitrobenzol in 200 ml Tetrahydrofuran gelöst, mit 3,1 g Pd/C (5 %ig) versetzt und 8 h bei 70°C und einem Wasserstoffdruck von 50 bar hydriert. Nach Filtration des Katalysators wird das Lösemittel im Vakuum abdestilliert und das Produkt durch Kristallisation aus Ethylacetat gereinigt. Das Produkt fällt mit 20 g als farbloser bis bläulicher Feststoff in 37,6 % d. Th. an.

Die Reinigung kann auch durch Chromatographie an Kieselgel mit Hexan/Ethylacetat erfolgen.
¹H-NMR (300 MHz, CDCl₃): 3,67 (m, 2 H, C*H*₂CH₂), 3,99 (m, 2 H, CH₂C*H*₂), 4,27 (s, 2 H, C*H*₂CO), 6,68 (d, 2 H, ³*J*=8,71 Hz, C*H*CH), 7,03 (d, 2 H, ³*J*=8,71 Hz, CHC*H*)
MS (r.I.%) = 192 (100, M⁺˙), 163 (48), 133 (26), 119 (76), 106 (49), 92 (38), 67 (27), 65 (45), 52 (22), 28 (22)

### Synthesebeispiele

### Beispiel 44

### 5-Chloro-N-({(5S)-2-oxo-3-[4-(3-oxo-4-morpholinyl)phenyl]-1,3-oxazolidin-5-yl}methyl)-2-thiophencarboxamid

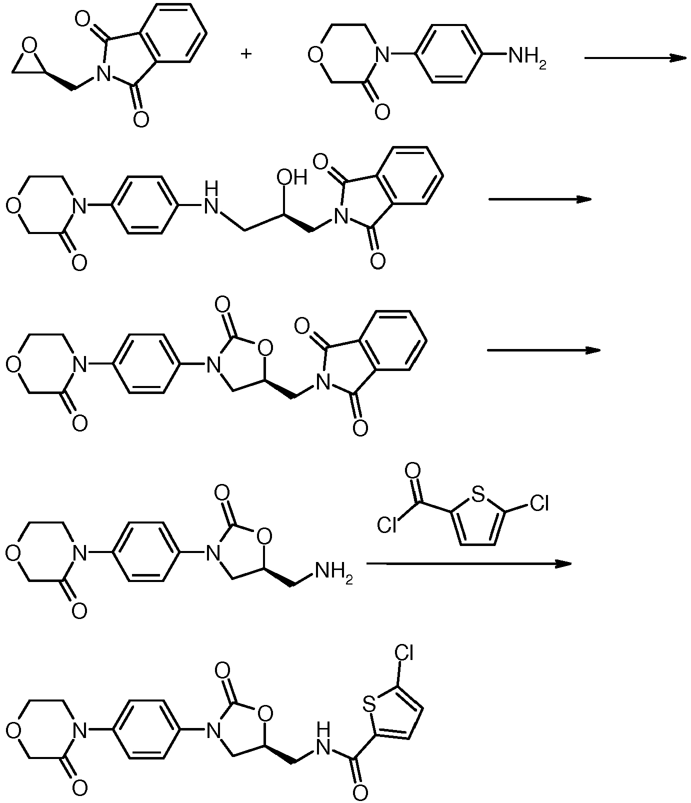

### a) 2-((2R)-2-Hydroxy-3-{[4-(3-oxo-4-morpholinyl)phenyl]amino}propyl)-1H-isoindol-1,3(2H)-dion:

Eine Suspension von 2-[(2*S*)-2-Oxiranylmethyl]-1*H*-isoindol-1,3(2*H*)-dion (A. Gutcait et al. Tetrahedron Asym. 1996, 7, 1641) (5.68 g, 27.9 mmol) und 4-(4-Aminophenyl)-3-morpholinon (5.37 g, 27.9 mmol) in Ethanol-Wasser (9:1, 140 ml) wird für 14 h refluxiert (der Niederschlag geht in Lösung, nach einiger Zeit erneute Bildung eines Niederschlages). Der Niederschlag (gewünschtes Produkt) wird abfiltriert, dreimal mit Diethylether gewaschen und getrocknet. Die vereinigten Mutterlaugen werden im Vakuum eingeengt und nach Zugabe einer zweiten Portion 2-[(2*S*)-2-Oxiranylmethyl]-1*H*-isoindol-1,3(2*H*)-dion (2.84 g, 14.0 mmol) in Ethanol-Wasser (9:1, 70 ml) suspendiert und für 13 h refluxiert (der Niederschlag geht in Lösung, nach einiger Zeit erneute Bildung eines Niederschlages). Der Niederschlag (gewünschtes Produkt) wird abfiltriert, dreimal mit Diethylether gewaschen und getrocknet. Gesamtausbeute : 10.14 g, 92 % der Theorie.
MS (ESI): m/z (%) = 418 ([M+Na]⁺, 84), 396 ([M+H]⁺, 93);
HPLC (Methode 3): rt (%) = 3.34 (100).

### b) 2-({(5S)-2-Oxo-3-[4-(3-oxo-4-morpholinyl)phenyl]-1,3-oxazolidin-5-yl}methyl)-1H-isoindol-1,3(2H)-dion:

Zu einer Suspension des Aminoalkohols (3.58 g, 9.05 mmol) in Tetrahydrofuran (90 ml) wird unter Argon bei Raumtemperatur *N*,*N'*-Carbonyldiimidazol (2.94 g, 18.1 mmol) und Dimethylaminopyridin (katalytische Menge) gegeben. Die Reaktionssuspension wird bei 60°C für 12 h gerührt (der Niederschlag geht in Lösung, nach einiger Zeit erneute Bildung eines Niederschlages), mit einer zweiten Portion *N*,*N'*-Carbonyldiimidazol (2.94 g, 18.1 mmol) versetzt und weitere 12 h bei 60°C gerührt. Der Niederschlag (gewünschtes Produkt) wird abfiltriert, mit Tetrahydrofuran gewaschen und getrocknet. Das Filtrat wird im Vakuum eingeengt und weiteres Produkt mittels Flash-Chromatographie (Dichlormethan-Methanol-Gemische) gereinigt. Gesamtausbeute: 3.32 g, 87 % der Theorie.
MS (ESI): m/z (%) = 422 ([M+H]⁺, 100);
HPLC (Methode 4): rt (%) = 3.37 (100).

### c) 5-Chloro-N-({(5S)-2-oxo-3-[4-(3-oxo-4-morpholinyl)phenyl]-1,3-oxazolidin-5-yl}methyl)-2-thiophencarboxamid:

Zu einer Suspension des Oxazolidinons (4.45 g, 10.6 mmol) in Ethanol (102 ml) wird bei Raumtemperatur tropfenweise Methylamin (40%ig in Wasser, 10.2 ml, 0.142 mol) gegeben. Die Reaktionsmischung wird für 1 h refluxiert und im Vakuum eingeengt. Das Rohprodukt wird ohne weitere Reinigung in die nächste Reaktion eingesetzt.

Zu einer Lösung des Amins in Pyridin (90 ml) wird unter Argon bei 0°C 5-Chlorthiophen-2-carbonsäurechlorid (2.29 g, 12.7 mmol) getropft. Die Eiskühlung wird entfernt und das Reaktionsgemisch 1 h bei Raumtemperatur gerührt und mit Wasser versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Dichlormethan-Methanol-Gemische) gereinigt. Gesamtausbeute: 3.92 g, 86 % der Theorie.
Smp: 232-233°C;
¹H NMR (DMSO-d⁶, 200 MHz): 9.05-8.90 (t, *J* = 5.8 Hz, 1H), 7.70 (d, *J* = 4.1 Hz, 1H), 7.56 (d, *J* = 9.0 Hz, 2H), 7.41 (d, *J* = 9.0 Hz, 2H), 7.20 (d, *J* = 4.1 Hz, 1H), 4.93-4.75 (m, 1H), 4.27-4.12 (m, 3H), 4.02-3.91 (m, 2H), 3.91-3.79 (dd, *J* = 6.1 Hz, 9.2 Hz, 1H), 3.76-3.66 (m, 2H), 3.66-3.54 (m, 2H);
MS (ESI): m/z (%) = 436 ([M+H]⁺, 100, Cl-Muster);
HPLC (Methode 2): rt (%) = 3.60 (100);
[α]²¹D = -38° (c 0.2985, DMSO); ee: 99 %.
IC₅₀: 0.7 nM

### HPLC-Parameter und LC-MS Parameter der in den vorrangegangenen Beispielen angegebenen HPLC- und LC-MS-Daten (die Einheit der Retentionszeit (rt) ist Minuten):

[1] Säule: Kromasil C18, L-R Temperatur: 30°C, Fluss = 0.75 mlmin⁻¹, Eluent: A = 0.01 M HClO₄, B = CH₃CN, Gradient: -> 0.5 min 98%A -> 4.5 min 10%A ->6.5 min 10%A
[2] Säule: Kromasil C18 60*2, L-R Temperatur: 30°C, Fluss = 0.75 mlmin⁻¹, Eluent: A = 0.01 M H₃PO₄, B = CH₃CN, Gradient: -> 0.5 min 90%A -> 4.5 min 10%A ->6.5 min 10%A
[3] Säule: Kromasil C18 60*2, L-R Temperatur: 30°C, Fluss = 0.75 mlmin⁻¹, Eluent: A = 0.005 M HClO₄, B = CH₃CN, Gradient: -> 0.5 min 98%A -> 4.5 min 10%A ->6.5 min 10%A
[4] Säule: Symmetry C18 2.1x150 mm, Säulenofen: 50°C, Fluss = 0.6 mlmin⁻¹, Eluent: A = 0.6 g 30%ige HCl/l Wasser, B = CH₃CN, Gradient: 0.0 min 90%A -> 4.0 min 10%A ->9 min 10%A
[5] MHZ-2Q, Instrument Micromass Quattro LCZ
   Säule Symmetry C18, 50 mm x 2.1 mm, 3.5 µm, Temperatur: 40°C, Fluss = 0.5 ml min⁻¹, Eluent A = CH₃CN + 0.1% Ameisensäure, Eluent B = Wasser + 0.1% Ameisensäure, Gradient: 0.0 min 10% A -> 4 min 90% A -> 6 min 90% A
[6] MHZ-2P, Instrument Micromass Platform LCZ
   Säule Symmetry C18, 50 mm x 2.1 mm, 3.5 µm, Temperatur: 40°C, Fluss = 0.5 mlmin⁻¹, Eluent A = CH₃CN + 0.1% Ameisensäure, Eluent B = Wasser + 0.1% Ameisensäure, Gradient: 0.0 min 10% A -> 4 min 90% A -> 6 min 90% A
[7] MHZ-7Q, Instrument Micromass Quattro LCZ
   Säule Symmetry C18, 50 mm x 2.1 mm, 3.5 µm, Temperatur: 40°C, Fluss = 0.5 mlmin⁻¹, Eluent A = CH₃CN + 0.1% Ameisensäure, Eluent B = Wasser + 0.1% Ameisensäure, Gradient: 0.0 min 5% A -> 1 min 5% A -> 5 min 90% A -> 6 min 90% A

Bei den oben aufgeführten Strukturen, die den oder die Reste 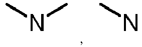 oder -O beinhalten, ist stets eine 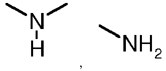 oder -OH-Funktion gemeint.

## Patentansprüche

1. Kombination mit synergistischer antithrombotischer Wirkung enthaltend
A) 5-Chloro-*N*-({(5*S*)-2-oxo-3-[4-(3-oxo-4-morpholinyl)phenyl]-1,3-oxazolidin-5-yl}methyl)-2-thiophencarboxamid der Formel 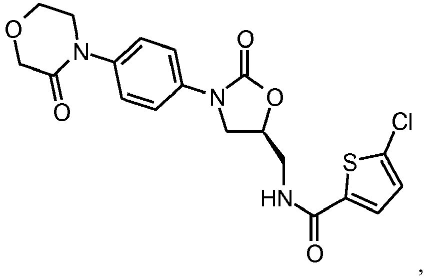
B) Acetylsalizylsäure
und
C) Clopidogrel.

2. Verfahren zur Herstellung einer Kombination mit synergistischer antithrombotischer Wirkung nach Anspruch 1, **dadurch gekennzeichnet, dass** man 5-Chloro-*N*-({(5*S*)-2-oxo-3-[4-(3-oxo-4-morpholinyl)phenyl]-1,3-oxazolidin-5-yl}methyl)-2-thiophencarboxamid, Acetylsalizylsäure und Clopidogrel in geeigneter Weise kombiniert oder herrichtet.

3. Kombination mit synergistischer antithrombotischer Wirkung nach Anspruch 1 zur Prophylaxe und/oder Behandlung von Erkrankungen.

4. Arzneimittel, enthaltend die Kombination mit synergistischer antithrombotischer Wirkung nach Anspruch 1 und gegebenenfalls weitere pharmazeutische Wirkstoffe.

5. Arzneimittel enthaltend die Kombination mit synergistischer antithrombotischer Wirkung nach Anspruch 1 sowie ein oder mehrere pharmakologisch unbedenkliche Hilfs- und/oder Trägerstoffe.

6. Verwendung einer Kombination mit synergistischer antithrombotischer Wirkung nach Anspruch 1 zur Herstellung eines Arzneimittels zur Prophylaxe und/oder Behandlung von thromboembolischen Erkrankungen.

7. Verwendung der Kombination mit synergistischer antithrombotischer Wirkung nach Anspruch 1 zur Herstellung eines Arzneimittels zur Prophylaxe und/oder Behandlung von Herzinfarkt mit ST-Segment-Erhöhung (STEMI) und ohne ST-Segment-Erhöhung (non-STEMI), stabiler Angina Pectoris, instabiler Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken, thrombotischem und thromboembolischem Hirnschlag.

## Claims

1. Combination with synergistic antithrombotic effect comprising
A) 5-chloro-*N*-({(5*S*)-2-oxo-3-[4-(3-oxo-4-morpholinyl)phenyl]-1,3-oxazolidin-5-yl}methyl)-2-thiophenecarboxamide of the formula 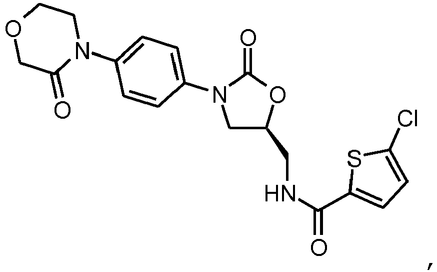
B) acetylsalicylic acid
and
C) clopidogrel.

2. Process for producing a combination with synergistic antithrombotic effect according to Claim 1, **characterized in that** 5-chloro-*N*-({(5*S*)-2-oxo-3-[4-(3-oxo-4-morpholinyl)phenyl]-1,3-oxazolidin-5-yl}methyl)-2-thiophenecarboxamide, acetylsalicylic acid and clopidogrel are combined or prepared in a suitable way.

3. Combination with synergistic antithrombotic effect according to Claim 1 for the prophylaxis and/or treatment of disorders.

4. Medicament comprising the combination with synergistic antithrombotic effect according to Claim 1 and, where appropriate, further active pharmaceutical ingredients.

5. Medicament comprising the combination with synergistic antithrombotic effect according to Claim 1 and one or more pharmacologically suitable excipients and/or carriers.

6. Use of a combination with synergistic antithrombotic effect according to Claim 1 for producing a medicament for the prophylaxis and/or treatment of thromboembolic disorders.

7. Use of the combination with synergistic antithrombotic effect according to Claim 1 for producing a medicament for the prophylaxis and/or treatment of myocardial infarction with ST segment elevation (STEMI) and without ST segment elevation (non-STEMI), stable angina pectoris, unstable angina pectoris, reocclusions and restenoses following coronary interventions such as angioplasty or aortocoronary bypass, peripheral arterial occlusive diseases, pulmonary embolisms, deep vein thromboses and renal vein thromboses, transient ischaemic attacks, and thrombotic and thromboembolic stroke.

## Revendications

1. Combinaison à effet antithrombotique synergique, contenant
A) le 5-chloro-*N*-({(5S)-2-oxo-3-[4-(3-oxo-4-morpholinyl)phényl]-1,3-oxazolidin-5-yl}méthyl)-2-thiophène-carboxamide de formule 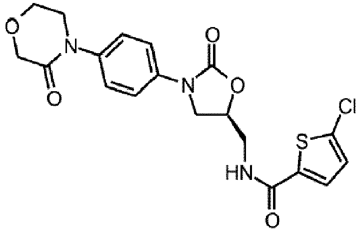
B) de l'acide acétylsalicylique,
et
C) du clopidogrel.

2. Procédé de fabrication d'une combinaison à effet antithrombotique synergique selon la revendication 1, **caractérisé en ce que** du 5-chloro-N-({(5S)-2-oxo-3-[4-(3-oxo-4-morpholinyl)phényl]-1,3-oxazolidin-5-yl}méthyl)-2-thiophène-carboxamide, de l'acide acétylsalicylique et du clopidogrel sont combinés ou préparés d'une manière appropriée.

3. Combinaison à effet antithrombotique synergique selon la revendication 1 pour la prophylaxie et/ou le traitement de maladies.

4. Médicament, contenant la combinaison à effet antithrombotique synergique selon la revendication 1 et éventuellement d'autres agents actifs pharmaceutiques.

5. Médicament, contenant la combinaison à effet antithrombotique synergique selon la revendication 1 et un ou plusieurs adjuvants et/ou véhicules pharmacologiquement sûrs.

6. Utilisation d'une combinaison à effet antithrombotique synergique selon la revendication 1 pour la fabrication d'un médicament pour la prophylaxie et/ou le traitement de maladies thromboemboliques.

7. Utilisation de la combinaison à effet antithrombotique synergique selon la revendication 1 pour la fabrication d'un médicament pour la prophylaxie et/ou le traitement de l'infarctus du myocarde avec élévation du segment ST (STEMI) et sans élévation du segment ST (non-STEMI), de l'angine de poitrine stable, de l'angine de poitrine instable, des réocclusions et des resténoses après des interventions coronariennes telles qu'une angioplastie ou un pontage aorto-coronarien, des artériopathies oblitérantes des membres inférieurs, des embolies pulmonaires, des thromboses veineuses profondes et des thromboses veineuses rénales, des attaques ischémiques transitoires, des attaques cérébrales thrombotiques et thromboemboliques.
